# EUROPEAN PATENT APPLICATION

(11) **EP 0 791 341 A1**
(43) Date of publication of application: **27.08.1997**
(21) Application number: 96200454.5
(22) Date of filing: 22.02.1996
(51) Int. Cl.: A61F 2/06

(54) **Wire stent**

(71) Applicant: N.V. BEKAERT S.A., 8550 Zwevegem (BE)
(72) Inventor: Demeyere, Eddy, 8510 Marke (BE); D'Haene, Urbain, 8570 Ingooigem (BE); Du Tre, Isaac, 9600 Ronse (BE)
(74) Representative: Ryckeboer, Leo L.H.

(57) **Abstract**

The invention relates to wire stents and methods of making them. The wire stent (1) comprises a wire structure defining a substantially circular cylindrical wall (2) wherein a number of consecutive substantially straight wire sections (3) follow at least one generator of the cylinder whereas the other sections of the structure are each shaped in substantially identical threedimensional closed loops (4) which are transversely oriented to said generator and fixed to it in points (5) at regular intervals thereby following approximately the circumference of the cylindrical wall, characterised in that each threedimensional loop (4) is obtained by bending a planar substantially elliptical loop to two planes around an axis which is substantially shorter than the long axis (7) of the ellipse.

## Description

The invention relates to a cylindrical wire stent for implantation into a body vessel and which is expandable by an angioplasty balloon associated with a catheter to dilate and expand the vessel, in particular the lumen of a blood vessel.

### Background of the invention.

Wire stents comprising a wire structure defining a cylindrical wall are known eg from U.S. patents 5 133 732, 5 161 547, 5 314 444 and 5 135 536. The starting product to make these stents is a wire shaped into a zig-zag pattern and further coiling or bending it to a tubular structure. Although these stent structures are generally easy to insert and have a relatively high expansion ratio and good visibility by eg X-rays, they may have an insufficient resistance to radial forces.

### Brief description of the invention.

It is an object of the invention to provide a wire stent with an improved resistance to radial forces in its final expanded form in the vessel. The stent according to the invention, and as supplied to the user for placement in the vessel, comprises a wire structure defining a substantially circular cylindrical wall wherein a number of consecutive substantially straight wire sections follow at least one generator of the cylinder whereas the other sections of the structure are each shaped in substantially identical threedimensional closed loops which are transversely oriented to said generator and fixed to it in points at regular intervals thereby following approximately the circumference of the cylindrical wall. The stent is characterised in that each threedimensional loop is obtained by bending a planar substantially elliptical loop to a number of planes around a number of different axes, preferably to two planes around an axis which is substantially shorter than the long axis of the ellipse. Said threedimensional loop is preferably obtained by bending said planar elliptical loop with one of its axes extending from said fixing point, to two planes around the short axis of the ellipse.

The wire for making the stent is preferably a stainless steel wire although Ta-, Ti-, Pt- or ELGILOY- wires are suitable as well. (Elgiloy is a registered trademark). It will preferably have a circular cross section and the wire diameter in the straight sections and in the loops is preferably the same. The wire diameter will preferably range between 2 % and 10 % of the equivalent diameter of a circular loop. The equivalent diameter means here the diameter of the circle with the same length of periphery as that of said planar elliptical loop.

It may be advantageous to use a crimped wire in the straight wire sections and/or in the wire loops. The wires are then preferably crimped with an amplitude of not more than two times the wire thickness, whereas the crimp wave length is between two and five times said amplitude.

The distance between each two consecutive fixing points ranges between 10 % and 100 % of said equivalent diameter and preferably between 20 % and 50 %. The wire stent is generally covered with a biocompatible substance such as heparin, DLC or certain polymers. The biocompatible substance is in particular a haemocompatible, antithrombogenic substance refraining any intimal hyperplasia.

It is also an object of the invention to provide some simple methods of making the wire stents described above.

In one method the stent is made from a continuous wire and comprises the steps of
a) bending said wire at regular intervals to thereby produce consecutive planar wire loops whereby all loops have substantially the same length and are linked to each other by intermediate straight wire sections ;
b) fixing the two ends of each of said loops to each other in fixing points ;
c) orienting the wire loop planes parallel to each other and transverse to said intermediate sections ;
d) optionally reshaping the loops in their plane to a geometrical shape so that the thus bended wire defines the surface of a cylindrical wall with a substantially elliptical cross section, and
e) bending each planar loop in a threedimensional shape around an axis which is substantially shorter than the long axis of the ellipse.

In step d one of the geometrical axes of the ellipse will preferably extend from each pair of fixed loop ends and the symmetrical bending of each said planar loop in a threedimensional shape is carried out around the short axis of the ellipse.

It is thereby possible to carry out step c before step b or together with step a.

The invention relates also to another manufacturing process which does not start with a continuous wire but with a set of wire rings and straight wires. This method as well as certain embodiments of stents according to the invention will be more fully described further with reference to the attached drawings.

### Brief description of the drawings.

Figure 1 is a perspective view of wire stent according to the invention.

Figure 2 is a cross sectional view of figure 1 and Figure 3 is a similar cross sectional view but wherein the elliptical loop is oriented in a different direction with respect to the straight wire sections.

Figure 4 shows a possible initial step of forming a wire stent structure when use is made of one continuous wire.

Figure 5 is a perspective view of the wire structure shown in figures 1 and 2 before the bending of the planar loops to a threedimensional shape.

Figure 6 is a similar view for a stent construction according to figure 3.

Figure 7 shows an intermediate wire structure, produced by suitably twisting and bending a continuous wire in view of obtaining the wire stent shown in Figure 8.

Figure 9 illustrates a set of wire rings arranged parallel to each other and to which one or two straight wires are fixed.

Figure 10 shows a wire stent produced from a crimped wire.

### Detailed description.

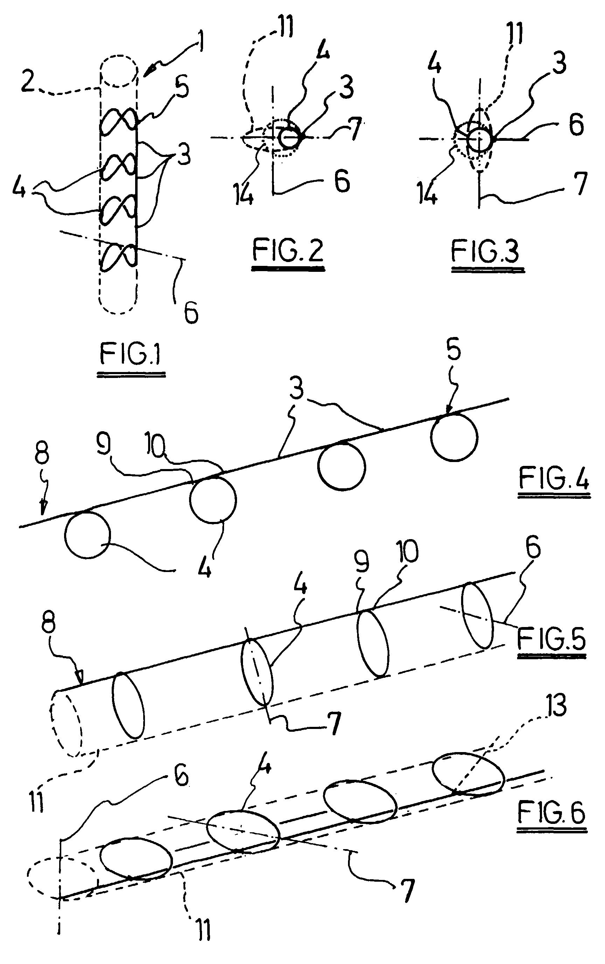

The wire stent 1 shown in Figure 1 comprises a wire structure defining a substantially circular cylindrical wall 2 wherein a number of consecutive substantially straight wire sections 3 follow one generator of the cylinder whereas the other sections of the structure are each shaped in substantially identical threedimensional closed loops 4 which are transversely oriented to said generator and fixed to it in points 5 at regular intervals thereby following approximately or substantially closely the circumference of the circular cylindrical wall. Each three dimensional loop 4 therein is obtained by symmetrically bending a planar substantially elliptical loop, with one of its axes 6, 7 extending from said fixing point 5 to two planes around the short axis 6 of the ellipse.

The distance between each two consecutive fixing points 5 ranges between 10 % and 100 % of the equivalent diameter of loops 4 (as defined hereinbefore) and is often between 20 % and 50 % of said diameter. This is desirable to arrange the consecutive loops 4 close enough to each other to approach a cylindrical wall with only very small circumferential gaps between the different wire loops.

To reach and retain an optimal stiffness in the wall structure the wire diameter should preferably range for certain stent designs (wire network patterns) between 2 % and 10 % and most preferably between 2 % and 6 % of said equivalent diameter of a circular loop.

In the embodiment of Figure 1 said consecutive sections 3 are aligned along one generator of said wall 2 thereby constituting a reinforcement of the structure and acting as a kind of backbone for the stent. This is advantageous in particular for the design of relatively long stent bodies as known eg from U.S. patent 5 133 732.

When making a stent starting from a continuous wire 8, the wire can in a first step be bent eg. as shown in Figure 4 at regular intervals to thereby produce consecutive planar wire loops 4 whereby all loops have substantially the same length and are linked to each other by intermediate straight wire sections 3. The two ends 9 and 10 of each of said loops are fixed to each other in fixing points 5, either by welding (eg TIG or MIG or resistance welding), by glueing, by soldering or mechanically by knotting, twisting or by otherwise clamping said ends to each other.

The wire loop ends can first be fixed to each other and the wire loop planes 4 can then be oriented parallel to each other and transverse to said intermediate sections 3 or vice versa. Actually it is possible and even preferred to carry out the different manufacturing steps in a (semi-) automatic manner. This can be done eg by suitably bending the leading end of the wire 8 to the desired substantially elliptical loop form around a mandrel (not shown), fixing the loop ends 9 and 10 to each other while on said mandrel and sliding the loop 4 axially over said mandrel in a forward movement over a distance equal to the length of a first section 3. Thereafter an identical second loop 4 is formed and the process is repeated to form a cylindrical wall 11 with a substantially elliptical cross section as shown in Figures 5 or 6. One of the geometrical axes 6, 7 of the ellipse can thereby extend from each pair of fixed loop ends 9, 10 (fixing point 5) as illustrated in Figures 2, 3, 5 and 6. The term "substantially elliptical" includes also oval, egg-shaped, ovoid, polygonal and rectangular with rounded off edges.

The final shaping step in making the stent comprises the symmetrical bending of each planar loop 4 into its three dimensional shape. This can eg be realised by putting the structure of Figure 5 or 6 inside a tubular caliber with the elliptical cross section 11 and with a suitable longitudinal slit in its wall. A comb is then radially inserted inwardly in this slit whereby the distance between adjacent comb teeth equals the axial length of the sections 3. The teeth of the comb coincide preferably with the short axis of the elliptical cross section. The caliber has a tapered outlet tube which gradually changes from the inside elliptical cross section 11 to the smaller ultimate circular cross section 2 (at its exit), desired for the stent wall. The elliptical stent with wall 11 is then advanced with the comb axially in the tubular cavity of the caliber to the outlet whereby the loops are gradually bent to their final threedimensional shape against the conically tapered inner wall of the outlet section of said caliber. The stent delivered at the outlet of the caliber defines the cylindrical wall 2.

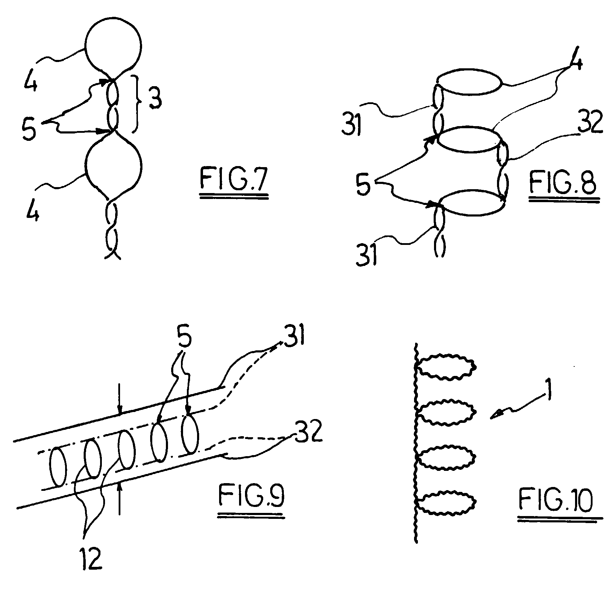

A part of said consecutive sections 3 can follow one generator 31 and the other sections then follow another generator 32, preferably situated at the side of wall 2 which is opposite to the place of said one generator 31. This is shown in the embodiments of Figures 8 and 9. According to the embodiment of Figure 8 the consecutive sections along generator 31 alternate over the length of the wall with the sections along the generator 32. Figure 7 shows a possible intermediate configuration which starts again from a continuous wire which is bent in a self explanatory manner to form twisted sections 3 linking consecutive loops 4. To arrive from the structure of Figure 7 to the stent of Figure 8, the loops 4 are oriented transversely to the sections 3 by bending them over 90° in the points 5.

As shown in Figure 9, in another method of making a wire stent 1 according to the invention a set of consecutive identical elliptical wire rings 12 are arranged parallel to each other (eg on a mandrel) at substantially equal distances to define a cylindrical wall 11. At least one wire 31 is fixed to said rings along a generator (31) contacting preferably consecutive ellipse apexes 5 of said rings. The rings 12 are further symmetrically bent in a threedimensional shape, preferably around the short axis 6 of the ellipse to form the cylindrical stent wall 2 as described above and shown in Figure 1. It is also possible to fix another wire 32 on the opposite apexes of the rings along a generator (32). It is in principle even possible to bend the rings 12 to their threedimensional shape around an axis 13 (figure 6) which does not coincide with the short axis 6, but which is substantially shorter than the long axis 7 of the ellipse.

The wires will preferably have a circular cross section and the wire diameter in the sections 3 and in the loops 4 is generally the same. However the wire diameter in sections 3 can either be smaller or larger than that of the loops 4.

### Example.

A round stainless steel wire of the type AISI 316 with a diameter of 0.2 mm is bent in the shape according to Figure 4. The length of each straight section 3 is chosen at 1.5 mm and the diameter of each circular loop 4 is set at 5 mm. The loop ends are fixed to each other by a TIG weld and the loops are oriented parallel to each other and transverse to the direction of the sections 3. The cylindrical structure has an axial length of eg 12 mm and is put in a pair of pliers with a suitable gap to reshape the cylindrical stent wall to one with an elliptical cross section to form the wall 11. The final step in the shaping can also be carried out in a caliber as described above. Each planar elliptical loop is thereby bent to its desired threedimensional shape as shown in Figure 1 to obtain a stent wall 2 with a diameter of about 2 mm. This tubular stent can be easily slid over the surface of a deflated angioplasty balloon. The stent is further compressed against the surface of this deflated balloon and inserted with the balloon (and catheter) in the vessel at the appropriate place. By inflating the balloon against the inner wall of the vessel, the stent is expanded to its initial shape with almost circular loops 14 as shown in Figures 2 and 3 with loop diameters up to 5 mm. The length of the loops 14 equals the length of the loops 11. Since the wire loops 4 are thereby brought back almost to their planar configuration, the resistance to radial compression forces is substantially improved.

The principle of making an expandable tubular wire structure according to the invention is also applicable for other purposes than for making and using a wire stent. Any tube with a flexible or elastic and collapsible wall, which has to sustain circumferential pressure from the outside, can internally be reinforced by introducing a wire structure according to the invention in its inside and expanding it against the inner wall of the tube.

Alternatively, a tube of highly stretchable material can be covered on its outer side with a wire structure according to the invention (eg as shown in figure 1). Under the application of internal pressure in the tube, it can be inflated and expanded. The tube with the surrounding wire structure then expands gradually up to the limit where the three dimensional wire loops stretch out to circular planar loops, thereby reinforcing the tube at its outer wall side.

## Claims

1. A wire stent (1) comprising a wire structure defining a substantially circular cylindrical wall (2) wherein a number of consecutive substantially straight wire sections (3) follow at least one generator of the cylinder whereas the other sections of the structure are each shaped in substantially identical threedimensional closed loops (4) which are transversely oriented to said generator and fixed to it in points (5) at regular intervals thereby following approximately the circumference of the cylindrical wall, characterised in that each threedimensional loop (4) is obtained by bending a planar substantially elliptical loop to two planes around an axis which is substantially shorter than the long axis (7) of the ellipse.

2. A wire stent according to claim 1 wherein each threedimensional loop is obtained by bending said elliptical loop, with one of its axes (6,7) extending from said fixing point (5), to two planes around the short axis (6) of the ellipse.

3. A wire stent according to claim 1 wherein the wire is a stainless steel wire with a diameter ranging between 2 % and 10 % of the equivalent diameter of a circular loop.

4. A wire stent according to claim 1 wherein the distance between each two consecutive fixing points ranges between 10 % and 100 % of said equivalent diameter.

5. A wire stent according to claim 4 wherein said distance ranges between 20 % and 50 % of said diameter.

6. A wire stent according to claim 1 wherein the wire is covered with a biocompatible substance.

7. A wire stent according to claim 1 wherein said consecutive sections (3) are aligned along one generator of said wall (2).

8. A wire stent according to claim 1 wherein a part of said consecutive sections (3) follow one generator (31) and the other sections follow another generator (32).

9. A wire stent according to claim 8 wherein said other generator (32) is situated at the side of said wall (2) which is opposite to the place of said one generator (31).

10. A wire stent according to claim 9 wherein the consecutive sections along said one generator (31) alternate over the length of the wall with the sections along said other generator (32).

11. A wire stent according to claim 1 comprising wires with a circular cross section and wherein the wire diameter in the sections (3) and in the loops (4) is the same.

12. A wire stent according to claim 1 wherein the wires in the straight wire sections (3) and/or in the wire loops (4) are crimped with an amplitude of not more than two times the wire thickness, whereas the crimp wave length is between two and five times said amplitude.

13. A method of making a wire stent (1) according to claim 1 from a continuous wire (8) comprising the steps of
a) bending said wire at regular intervals to thereby produce consecutive planar wire loops (4) whereby all loops have substantially the same length and are linked to each other by intermediate straight wire sections (3) ;
b) fixing the two ends (9) and (10) of each of said loops to each other in fixing points (5) ;
c) orienting the wire loop planes (4) parallel to each other and transverse to said intermediate sections ;
d) optionally reshaping the loops (4) in their plane to a geometrical shape so that the thus bent wire defines the surface of a cylindrical wall (11) with a substantially elliptical cross section and,
e) bending each planar loop (4) in a threedimensional shape around an axis which is substantially shorter than the long axis (7) of the ellipse.

14. A method of making a wire stent according to claim 13 wherein for step d) one of the geometrical axes (6, 7) of the ellipse extends from each fixing point (5) and wherein in step e) a symmetrical bending of each planar loop (4) in a threedimensional shape is carried out around the short axis (6) of the ellipse.

15. A method according to claim 13 wherein step c is carried out before step b.

16. A method according to claim 13 wherein step c is carried out together with step a.

17. A method of making a wire stent (1) according to claim 1 wherein
a) a set of consecutive identical elliptical wire rings (12) are arranged parallel to each other at substantially equal distances to define a cylindrical wall (11) and wherein
b) at least one wire is fixed to said rings along a generator (31) and wherein
c) the rings (12) are further bent in a threedimensional shape around an axis which is substantially shorter than the long axis (7) of the ellipse.

18. A method according to claim 17 wherein in step b at least one generator (31) contacts consecutive ellipse apexes (5) of said rings and wherein in step c) the rings (12) are further symmetrically bent in a threedimensional shape around the short axis (6) of the ellipse to form the cylindrical stent wall (2).
